# EUROPEAN PATENT APPLICATION

(11) **EP 1 375 462 A1**
(43) Date of publication of application: **02.01.2004**
(21) Application number: 02013677.6
(22) Date of filing: 20.06.2002
(51) Int. Cl.: C07C 41/42, C07C 41/36, C07C 43/13

(54) **Process of separating 1-methoxy-2-propanol and 2-methoxy-1-propanol from aqueous compositions**

(71) Applicant: Degussa AG, 40474 Düsseldorf (DE); Uhde GmbH, 44141 Dortmund (DE)
(72) Inventor: Hofen, Willi, 63517 Rodenbach (DE); Gehrke, Helmut, Dr., 59269 Beckum-Vellern (DE); Kolbe, Bärbel, Dr., 58452 Witten (DE); Wilken, Dieter, 48268 Greven (DE); Gehlen, Carsten, 45772 Marl (DE); Kampeis, Percy, Dr., 44287 Dortmund (DE)
(74) Representative: Sternagel, Fleischer, Godemeyer & Partner Patentanwälte

(57) **Abstract**

The present invention refers to process of separating 1-methoxy-2-propanol and 2-methoxy-1-propanol from aqueous compositions, comprising: dewatering of the aqueous composition comprising 1-methoxy-2-propanol and 2-methoxy-1-propanol to a concentration of 1-methoxy-2-propanol and 2-methoxy-1-propanol of at least 90 percent by weight in total and isolation of 1-methoxy-2-propanol, 2-methoxy-1-propanol or mixtures thereof by means of distillation.

## Description

The present invention refers to a process of separating 1-methoxy-2-propanol and 2-methoxy-1-propanol from aqueous compositions. 1-methoxy-2-propanol and 2-methoxy- 1- propanol are valuable compounds that can be used e.g. as a solvent. The aqueous composition from which 1-methoxy-2-propanol and 2-methoxy-1-propanol are separated may be a waste water stream that is generated in the production of propylene oxide.

Propylene oxide is a very important intermediate product in the chemical industry. It can be processed for instance to polyester polyols, that are raw materials for the production of polyurethanes. Other end products of propylene oxide are for instance glycol ethers and propylene glycols.

EP-B-0 100 119 refers to a process for the epoxidation of olefinic compounds in the presence of hydrogen peroxide and a synthetic zeolite containing titanium atoms. In one embodiment propylene is reacted as olefinic compound in the presence of methanol as solubility promoter with said catalyst and hydrogen peroxide. Propylene oxide is obtained as desired reaction product and in addition to propylene oxide, glycol monomethylethers and propanediol are formed as by-products.

EP-B-0 100 118 discloses a single-step process for synthesizing glycol monomethylethers starting from an olefinic compound, methanol and hydrogen peroxide in an aqueous reaction medium. In order to conduct this reaction a catalytic amount of titanium silicalite is present in the reaction mixture. If propylene is used as olefinic compound the reaction leads to the formation of methoxy propanol. There is no indication which of the two possible isomeric methoxy propanols are produced and in what way they can be separated from the reaction mixture.

W0 98/47845 refers to a further single-step method for producing glycol monoethers from olefins. If said olefin is propylene, a reaction mixture is obtained in that methoxy propanol is the main product. Again, there is no indication which of the two possible isomers of methoxy propanol are formed and how they can be separated from the reaction mixture.

EP-B-0 425 893 discloses the separation of a mixture of 1-methoxy-2-propanol and water resulting from recrystallisation processes using 1-methoxy-2-propanol as solvent whereby the concentration of 1-methoxy-2-propanol is at least 55 percent by weight. The separation is conducted by means of pervaporation. The mixture of said compounds is contacted in liquid or gaseous phase with a hydrophilic membrane. The separation procedure is either a batch or a continuous process. Said process is only disclosed for a mixture consisting of water and 1-methoxy-2-propanol without any further compounds. The pervaporation method was chosen, because other dewatering processes like distillation or rectification failed in separating a mixture of 1-methoxy-2-propanol and water, especially azeotropic distillation methods were not successful.

The object of the present invention is to provide an economical process of separating 1-methoxy-2-propanol and 2-methoxy-1-propanol from aqueous compositions, whereby the isolated 1-methoxy-2-propanol, 2-methoxy-1-propanol or the mixture thereof has a purity that makes it useful for other applications even if the concentration of these valuable products in the initial aqueous composition is as low as 0,5 percent by weight.

The object is attained by a process of separating 1-methoxy-2-propanol and 2-methoxy-1-propanol from aqueous compositions comprising:
dewatering of the aqueous composition comprising 1-methoxy-2-propanol and 2-methoxy-1-propanol to a concentration of 1-methoxy-2-propanol and 2-methoxy-1-propanol of at least 90 percent by weight in total and isolation of 1-methoxy-2-propanol, 2-methoxy-1-propanol or mixtures thereof by means of distillation.

In a preferred embodiment the aqueous composition may be concentrated prior to the dewatering step a) to contain at least 10 percent by weight in total of 1-methoxy-2-propanol and 2-methoxy-1-propanol by means of distillation.

The aqueous composition used for the process of the present invention can be a waste water stream obtained from a process for the production of propylene oxide by reaction of propylene and hydrogen peroxide in the presence of a catalyst and methanol.

The separation of water from the aqueous composition can be conducted by any dewatering method. The dewatering step leads to a concentration of 1-methoxy-2-propanol and 2-methoxy-1-propanol of at least 90 percent by weight in total. Thereby at least 70 percent of the amount of water contained in the aqueous composition fed to the dewatering step a) is removed. It is even more preferred that at least 90 percent, more preferred at least 95 percent and in a most preferred embodiment at least 99 percent of the water in the initial aqueous composition is removed by means of dewatering.

The aqueous composition comprising the isomers of methoxy propanol fed to the dewatering step preferably contains the isomers in an amount of at least 10 weight percent in total and less than 55 weight percent, preferably less than 30 weight percent, most preferred less than 25 weight percent.

In one embodiment the dewatering can be conducted by means of azeotropic distillation using an additive, that forms a heteroazeotrope with water with a boiling point minimum. Preferably, the additive has a boiling point of less than 120°C. Suitable additives can be selected from the group consisting of aromatic hydrocarbons, paraffinic hydrocarbons, naphthenic hydrocarbons, halogenated hydrocarbons and mixtures thereof.

The aromatic hydrocarbons may be selected from benzene, toluene or mixtures thereof.

The paraffinic hydrocarbons may be selected from hexane, cyclohexane, heptane or mixtures thereof.

A suitable halogenated hydrocarbon may be selected from the group consisting of CHₓCl₄₋ₓ with x=0 to 3, C₂HₓCl₆₋ₓ with x=0 to 5, tetrachloroethylene and mixtures thereof.

The azeotropic distillation is conducted in a way that the amount of water and additive forming an azeotrope with water in the bottom stream of the distillation column is less than 1 percent by weight, preferably less than 0.1 percent by weight and most preferably less than 0.02 percent by weight in total.

According to an alternative embodiment dewatering of the aqueous composition comprising 1-methoxy-2-propanol and 2-methoxy-1-propanol can be achieved by extractive distillation. The extractive distillation can be conducted by adding an extraction solvent changing the volatility of water relative to 1-methoxy-2-propanol and 2-methoxy-1-propanol. Preferably, the extraction solvent has a boiling point of at least 130°C. Extraction solvent are suitable that either enhance the volatility of water relative to 1-methoxy-2-propanol and 2-methoxy-1-propanol or reduce the volatility of water relative to said compounds.

The preferred extraction solvent are dialcohols having the general formula CₙH₂ₙ(OH)₂ and n>1, such as ethylene glycol, propylene glycol, diethylene glycol, triethylene glycol, tetraethylene glycol or mixtures thereof.

Alternatively, dewatering of the aqueous composition can be achieved by a membrane separation. Thereby the aqueous composition comprising 2-methoxy-1-propanol and 1-methoxy-2-propanol is contacted with a semipermeable, hydrophilic membrane in a suitable apparatus either in liquid phase as pervaporation step or in the gaseous phase as vapor permeation step. Across the semipermeable membrane a pressure difference is established. The permeate will substantially contain water and only a minor amount of 1-methoxy-2-propanol and 2-methoxy-1-propanol. Accordingly the major amount of 1-methoxy-2-propanol and 2-methoxy-1-propanol fed to the apparatus will not pass the membrane and can be collected as a retentate with a reduced water content.

In a preferred embodiment the dewatering step a) can be a combination of a membrane separation step and a distillation step. Thereby a membrane separation using a small membrane unit is combined with a subsequent distillation unit to achieve substantially complete dewatering of the aqueous composition comprising 2 -methoxy- 1-propanol and 1 -methoxy-2-propanol. In this event, conditions for the membrane separation are selected in a way to give a retentate having a water content in the range of 5 to 25 percent by weight. This retentate is fed to the distillation column and separated to a distillate having a water content in the range of 30 and 50 percent by weight and a bottom product having a water content of less than 1 percent by weight, preferably less than 0.1 percent by weight. The distillate from said distillation column is either separately or mixed with the aqueous composition comprising 2-methoxy-1-propanol and 1-methoxy-2-propanol again fed to the membrane separation unit.

Subsequent to the dewatering step a), the mixture comprising substantially 1-methoxy-2-propanol and 2-methoxy-1-propanol is separated by means of distillation. Any distillation column known to be suitable to a person skilled in the art can be used for conducting the distillation. Said mixture of methoxy propanol isomers is separated in a distillate that substantially consists of 1-methoxy-2-propanol and a bottom product that substantially consists of 2-methoxy-1-propanol. The separation is preferably conducted at a pressure in the range of 0.5 to 5 bar and it is most preferred that the pressure is atmospheric pressure. The distillation columns used for this distillation method have generally a separation capability of at least 20 theoretical stages, whereby the enriching section has a higher separation capability than the stripping section. The column efficiency of the enriching section is preferably at least twice as much as the column efficiency of the stripping section. The reflux ratio is generally in the range of 5 to 50 and it is preferred that the reflux ratio is in the range of 8 to 12.

Said distillation can advantageously be conducted in a dividing-wall column. This is a distillation column, that is separated by means of a partition, located in the middle part of the column, in two separate areas that are connected at the upper and lower end with the same area of the distillation column, in a way that gas and liquids can pass from one area to another. The mixture obtained in the dewatering step is fed to the dividing-wall column on one side of the partition. On the other side of the partition,1-methoxy-2-propanol is obtained as a side stream, whereas low boiling impurities and residual water are removed with the distillate and 2-methoxy-1-propanol and high boilers are removed as bottom product. Thereby low boiling impurities and water can be separated from 1-methoxy-2-propanol. Thus, a subsequent distillation of 1-methoxy-2-propanol is not necessary.

The process of the present invention can be preferably applied to the separation of 1-methoxy-2-propanol and 2-methoxy-1-propanol from waste water streams resulting from the propene oxide production. Such waste water streams comprise 1,2-propane diol and carboxylic acids, such as formic acid, acetic acid and propionic acid, in addition to 1-methoxy-2-propanol and 2-methoxy-1-propanol. A typical composition of a waste water stream obtained from the epoxidation of propene using hydrogen peroxide in presence of methanol and a catalyst is as follows:

| | |
|---|---|
| 1-methoxy-2-propanol | 1 to 10 wt-% |
| 2-methoxy-1-propanol | 1 to 10 wt-% |
| formic acid | 0,001 to 3 wt-% |
| acetic acid | 0,001 to 3 wt-% |
| traces of propionic acid 1,2-propane diol | 0,1 to 10 wt-% |
| high boiling compounds (b.p. > 140°C) | 0,001 to 5 wt-% |

The pH value of such a composition is normally in the range of 2 to 5.

In a preferred embodiment of the invention, the aqueous composition is concentrated prior to the dewatering step a) by means of a pre-distillation to contain at least 10 percent by weight in total of 1-methoxy-2-propanol and 2-methoxy-1-propanol. The distillate obtained by such a distillation preferably contains the methoxy propanol isomers in an amount of 10 to 50 percent by weight. If dewatering is achieved by azeotropic distillation or extractive distillation, distillation conditions are preferably chosen to give a methoxy propanol content of 10 to 30 percent by weight and most preferred 10 to 25 percent by weight. If dewatering is achieved by membrane separation, distillation conditions are preferably chosen to give a methoxy propanol content of 20 to 50 percent by weight. The amount of 1-methoxy-2-propanol in the distillate is preferably at least 95 percent and most preferably at least 97 percent of the amount of the same in the aqueous composition fed to the distillation step.

Any suitable distillation column can be used for the above pre-distillation step. Preferably, the distillation column has at least 4 theoretical stages in the enriching section and at least 6 theoretical stages in the stripping section. The distillation column can be run at any suitable pressure. It is preferred that the pressure is in the range of 0.5 to 5 bar. Atmospheric pressure is most preferred.

If the aqueous composition is concentrated prior to the dewatering step a) by means of a pre-distillation, smaller equipment can be used and less energy is consumed for dewatering step a).

In addition to concentrating the methoxy propanol isomers, the pre-distillation efficiently removes 1,2-propane diol and high boiling impurities and reduces the amount of carboxylic acids to less than 1 percent. In a preferred embodiment of the present invention, the pH-value of the aqueous composition is adjusted prior feeding to the pre-distillation by adding a non-volatile base to at least 6, preferably to at least 8, more preferred to at least 10. Thereby, the total amount of carboxylic acids in the distillate can be further reduced to 0.001 to 0.1 percent by weight.

The pre-distillation prior to the azeotropic distillation or extractive distillation leads to a lower accumulation of impurities in the additive or extraction solvent recycle of said dewatering step and therefore less effort is necessary to remove said impurities, i.e. by purging and purifying the used additives to some extent. The pre-distillation prior to the dewatering by means of membrane separation leads to less fouling of the membrane and therefore permeation rates and membrane lifetime are increased.

In a preferred embodiment of the present invention, pre-distillation and dewatering by means of vapor permeation are combined so that the vapor obtained at the top of the pre-distillation column is contacted without prior condensation in gaseous phase with the semipermeable membrane. Combining pre-distillation and vapor permeation in such way saves-energy and requires less equipment compared to a conventional pervaporation unit.

In another embodiment of the present invention, a non-volatile base is added between the pre-distillation step and the dewatering step to adjust the pH-value of the aqueous composition fed to the dewatering step to at least 6, preferably to at least 8, more preferred to at least 10. Thus, less base is needed in comparison to adding the base prior to the pre-distillation step.

The present invention will be explained in more detail with reference to the figures. Auxilliary equipment like pumps, heat exchangers, vaporizers, condensers, etc. has been omitted from the drawings to enhance clarity.
Figure 1 exhibits a flow diagram of a preferred embodiment of the present invention using a pre-distillation.
Figure 2 shows one embodiment for the dewatering step a) using azeotropic distillation.
Figure 3 shows an alternative embodiment for the dewatering step a) using extractive distillation with an additive that reduces the relative volatility of water.
Figure 4 shows an alternative embodiment for the dewatering step a) using extractive distillation with an additive that increases the relative volatility of water.
Figure 5 shows a further embodiment for the dewatering step a) using membrane separation.
Figure 6 shows a preferred embodiment of the present invention using a membrane separation combined with a subsequent distillation for the dewatering step.
Figure 7 exhibits a dividing-wall column for separating 1-methoxy-2-propanol and 2-methoxy-1-propanol.

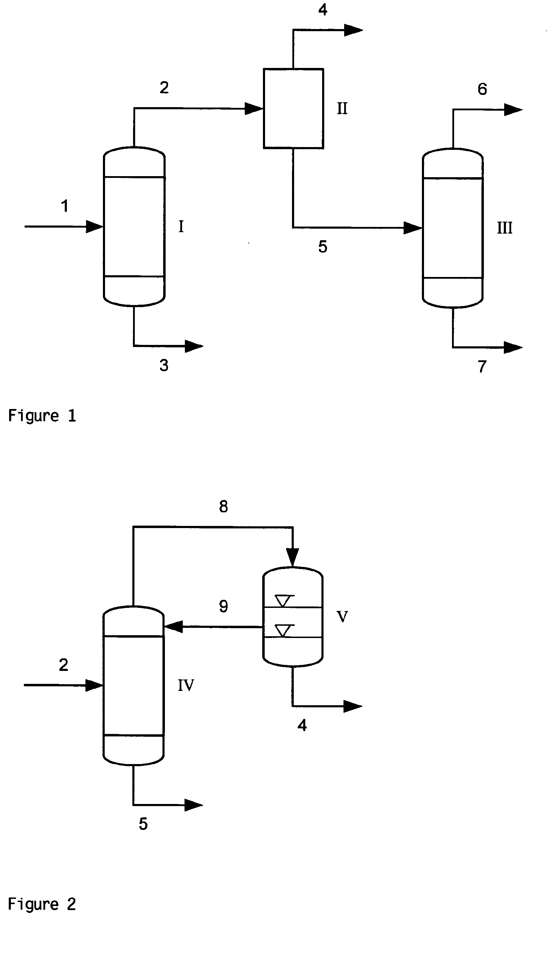

Referring to Fig.1. according to a preferred embodiment of the present invention an aqueous composition (1) comprising the isomers of methoxy propanol is fed to a distillation column (I). The distillate (2) comprising the majority of the methoxy propanol isomers is fed to a dewatering unit (II) where an aqueous stream (4) is withdrawn. Stream (5) comprising the majority of the methoxy propanol isomers and depleted of water is fed to a distillation column III and separated to a distillate stream (6) consisting essentially of 1-methoxy-2-propanol and a bottom stream (7) consisting essentially of 2-methoxy-1-propanol.

Figure 2 shows a unit for dewatering the aqueous composition comprising 1-methoxy-2-propanol and 2-methoxy-1-propanol by means of azeotropic distillation. The aqueous composition (2) and the azeotropic distillation agent (9) are fed to the azeotropic distillation column (IV). The distillate comprising the heteroazeotrope (8) is transferred to a phase separation vessel (V), where said distillate separates to a water phase (4), which is withdrawn, and a phase comprising essentially azeotropic distillation agent (9), which is fed to the azeotropic distillation column (IV). The dewatered mixture (5) of 1-methoxy-2-propanol and 2-methoxy-1-propanol is transferred to the distillation unit (III).

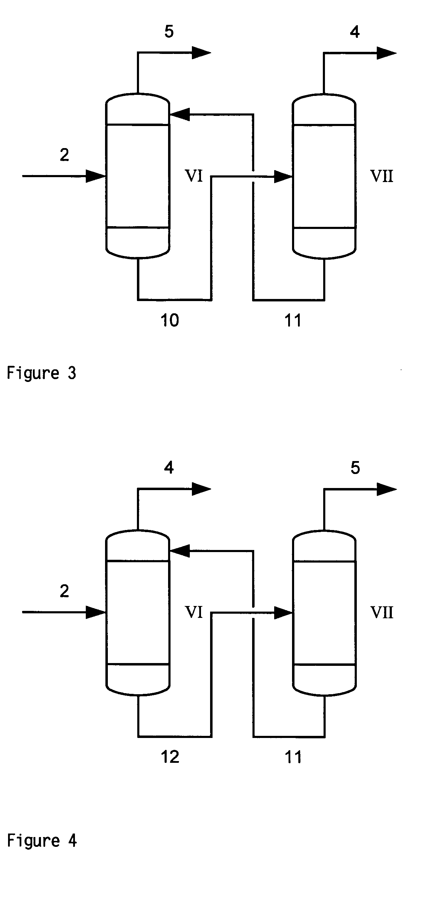

Figure 3 shows a unit for dewatering the aqueous composition comprising 1-methoxy-2-propanol and 2-methoxy-1-propanol by means of extractive distillation using an extraction solvent that decreases the relative volatility of water. The aqueous composition (2) and the extraction solvent (11) are fed to the extractive distillation column (VI). The dewatered mixture (5) of 1-methoxy-2-propanol and 2-methoxy-1-propanol is obtained as head product and transferred to the distillation unit (III). The bottom product (10) comprising water and the extraction solvent is transferred to a distillation column (VII) for recovering the extraction solvent (11). Water (4) is removed as the distillate from the distillation column (VII).

Figure 4 shows a unit for dewatering the aqueous composition comprising 1-methoxy-2-propanol and 2-methoxy-1-propanol by means of extractive distillation using an extraction solvent that enhances the relative volatility of water. The aqueous composition (2) and the extraction solvent (11) are fed to the extractive distillation column (VI). The head product (4) is essentially comprised of water. The bottom product is (12) essentially comprised of 1-methoxy-2-propanol, 2-methoxy-1-propanol and extraction solvent is transferred to distillation column (VII), where said bottom product is separated to a bottom product(11) comprising extraction solvent and a head product (5) comprising 1-methoxy-2-propanol and 2-methoxy-1-propanol. The extraction solvent (11) is reused and the mixture comprising 1-methoxy-2-propanol and 2-methoxy-1-propanol (5) is transferred to the distillation unit (III).

As alternative to the two separate distillation columns for conducting the extractive distillation as exhibited in Figure 4, a dividing-wall column such as described in EP 0 133 510 or WO 00/25881 can be utilized.

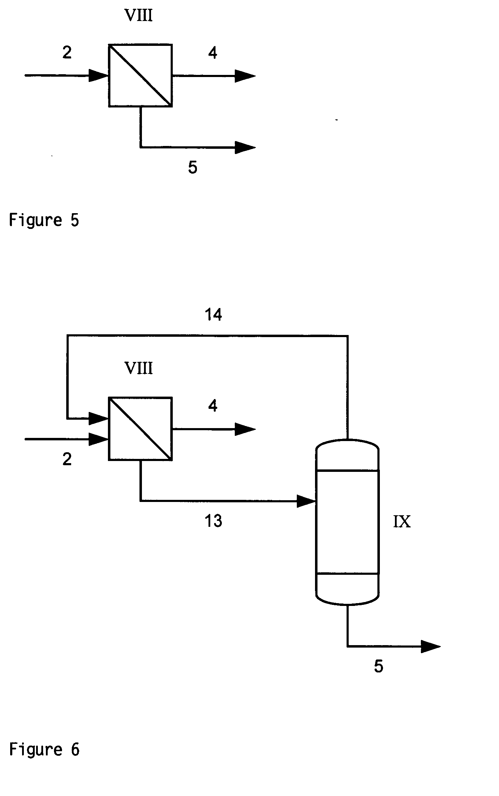

Figure 5 shows a means of dewatering the aqueous composition comprising 1-methoxy-2-propanol and 2-methoxy-1-propanol by means of membrane separation. The aqueous composition (2) comprising 1-methoxy-2-propanol and 2-methoxy-1-propanol is contacted with a semipermeable membrane in a membrane unit (VIII). The permeate (4) crossing the membrane is essentially comprised of water. The product stream (5), which is withheld by the membrane, substantially consists of 1-methoxy-2-propanol and 2-methoxy-1-propanol. Said product stream (5) is transferred to distillation unit (III).

Figure 6 shows a unit for dewatering, combining a membrane separation with a subsequent distillation step. The aqueous composition (2) comprising 1-methoxy-2-propanol and 2-methoxy-1-propanol is contacted with a semipermeable membrane in a membrane unit (VIII). The permeate (4) crossing the membrane is essentially comprised of water. The retentate stream (13), which is withheld by the membrane, is substantially comprised of 1-methoxy-2-propanol and 2-methoxy-1-propanol having a reduced water content and said retentate stream (13) is transferred to distillation column (IX). At the bottom of the distillation column a product stream (5) is obtained, that essentially consists of 1-methoxy-2-propanol and 2-methoxy-1-propanol having a minor water content. For subsequent separation said product stream is transferred to distillation unit (III). The head product (14) of column (IX) having an increased water content compared to column feed (13) is reused and fed to membrane unit (VIII).

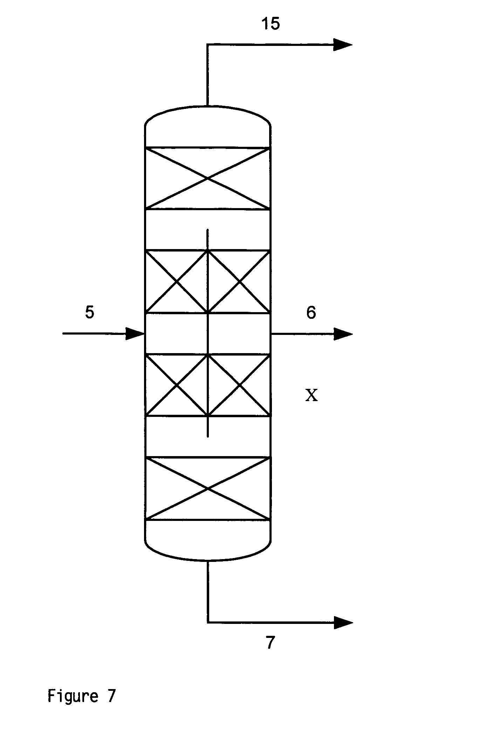

Figure 7 shows a dividing-wall column (X) as single distillation unit for separating 1-methoxy-2-propanol and 2-methoxy- 1-propanol from mixtures thereof containing residual water. The mixture (5) containing 1-methoxy-2-propanol, 2-methoxy-1-propanol and possibly residual water is fed to the distillation column. 1-methoxy-2-propanol (6) is removed as side stream from the column. The bottom product (7) essentially contains 2-methoxy-1-propanol. Residual water and low boiling impurities are contained in the head product (15).

The present invention is illustrated by the following examples without being limited to them.

### Examples

### Example 1:

The aqueous composition used for the process of separating 1-methoxy-2-propanol and 2-methoxy-1-propanol is a waste water stream obtained from a process of producing propylene oxide from propylene and hydrogen peroxide in the presence of methanol. The composition is given in table 1. The pH-value is 3.

**Table 1:**

| Component | percent by weight |
|---|---|
| 1-methoxy-2-propanol | 4,21 |
| 2-methoxy-1-propanol | 3,99 |
| 1,2-propane diol | 2,76 |
| formic acid | 1,98 |
| acetic acid | 0,18 |
| propionic acid | < 0,01 |
| dimethoxy methane | < 0,01 |
| 2-propanol | < 0,01 |
| water | rest |

The aqueous composition of table 1 was continuously fed to the 7th plate (counted from the top) of a bubble-cap column having 13 plates in total.

The feed rate was 307 g/h. The reboiler was heated so that at a reflux rate of 1,0, 84 g/h distillate was obtained at the top of the column. Additionally 223 g/h of bottom product was obtained. After establishing equilibrium in the distillation column, the distillate had a composition as indicated in table 2.

**Table 2:**

| composition of the distillate of example 1 | |
|---|---|
| Component | percent by weight |
| 1-methoxy-2-propanol | 13,8 |
| 2 - methoxy- 1 - propanol | 9,19 |
| 1,2-propane diol | < 0,01 |
| formic acid | 0,22 |
| acetic acid | < 0,01 |
| propionic acid | < 0,01 |
| dimethoxymethane | < 0,01 |
| 2-propanol | < 0,01 |
| water | rest |

The amount of 1-methoxy-2-propanol and 2-methoxy-1-propanol could significantly be increased by the pre-distillation step. The amount of formic acid was decreased, and acetic acid and propionic acid were nearly completely removed.

### Example 2:

Example 1 was repeated with the difference, that the pH-value of the aqueous composition was adjusted to 11 by adding sodium hydroxide prior to feeding said aqueous composition to the distillation column. After establishing equilibrium in the distillation column, the distillate had a composition as indicated in table 3.

**Table 3:**

| composition of the distillate of example 2 | |
|---|---|
| Component | percent by weight |
| 1-methoxy-2-propanol | 14,0 |
| 2-methoxy-1-propanol | 9,32 |
| 1,2-propane diol | < 0,01 |
| formic acid | not detectable |
| acetic acid | not detectable |
| propionic acid | not detectable |
| dimethoxymethane | < 0,01 |
| 2-propanol | < 0,01 |
| water | Rest |

Again, the amount of 1-methoxy-2-propanol and 2-methoxy-1-propanol could significantly be increased by pre-distillation. Formic acid, acetic acid and propionic acid were not detectable in the distillate.

### Example 3:

The distillate of example 2 was dewatered by means of azeotropic distillation using a distillation column having an inner diameter of 25 mm and a filling with stainless steel coils. The column had a separation efficiency of 25 theoretical stages and was equipped with external electric heating to compensate heat loss. The distillate of example 2 was fed continuously at a rate of 150 g/h to the 3rd theoretical stage (counted from the top) of said column. Additionally benzene was fed to the top of the column. The reboiler was heated to obtain 1235 g/h distillate. The distillate was condensed and transferred to a phase separation vessel, in that it separated to a benzene phase and an aqueous phase. The benzene phase was again fed to the top of the column and the aqueous phase was removed at a rate of 115 g/h. Simultaneously, a bottom product was removed at a rate of 35 g/h. The content of organic compounds of the bottom product was analyzed via gc-analysis. The water content was determined by Karl-Fischer-Titration.

**Table 4:**

| composition of the bottom product of example 3 | |
|---|---|
| Component | percent by weight |
| 1-methoxy-2-propanol | 60 |
| 2-methoxy- 1-propanol | 40 |
| 1,2-propane diol | not detectable |
| formic acid | not detectable |
| acetic acid | not detectable |
| propionic acid | not detectable |
| dimethoxymethane | not detectable |
| 2-propanol | not detectable |
| water | < 0,0001 |

Table 4 shows that the bottom product was a mixture of 1-methoxy-2-propanol and 2-methoxy-1-propanol with a very low content of water. Other impurities could not be detected.

### Example 4:

The bottom product of example 3 was fed continuously to the middle of a glass column with a length of 3000 mm packed with 3 mm stainless steel coils at a feed rate of 55 g/h. Heating was adjusted to obtain 33 g/h distillate at a reflux ratio of 6,2. Simultaneously, 22 g/h of bottom product were removed from the distillation column.

**Table 5:**

| composition of the distillate of example 4 | |
|---|---|
| Component | percent by weight |
| 1-methoxy-2-propanol | 99,0 |
| 2-methoxy-1-propanol | 1,0 |
| water | < 0,01 |
| acid content | not detectable |

Table 5 shows that the amount of 1-methoxy-2-propanol was increased to 99 percent by weight. The obtained 1-methoxy-2-propanol is essentially free of water and only a trace of 2-methoxy-1-propanol was present.

## Claims

1. A process of separating 1-methoxy-2-propanol and 2-methoxy-1-propanol from aqueous compositions, comprising:
a) dewatering of the aqueous composition comprising 1-methoxy-2-propanol and 2-methoxy-1-propanol to a concentration of 1-methoxy-2-propanol and 2-methoxy-1-propanol of at least 90 percent by weight in total, and
b) isolation of 1-methoxy-2-propanol and/or 2-methoxy-1-propanol or mixtures thereof from the product of step a) by means of distillation.

2. The process according to claim 1, **characterized in that** the aqueous composition is a waste water stream obtained from the production of propylene oxide by reaction of propylene and hydrogen peroxide in the presence of methanol.

3. The process according to any of the preceding claims, **characterized in that** the concentration of 1-methoxy-2-propanol and 2-methoxy-l-propanol in the aqueous composition is less than 55 percent by weight in total.

4. The process according to any of the preceding claims, **characterized in that** the aqueous composition is concentrated prior to dewatering step a) to contain 1-methoxy-2-propanol and 2-methoxy-1-propanol in an amount of at least 10 percent by weight in total by means of a pre-distillation.

5. The process according to any of the preceding claims, **characterized in that** prior to the dewatering step a), the pH of said aqueous composition is adjusted to be at least 6.

6. The process according to claim 5, **characterized in that** prior to the pre-distillation step, the pH of said aqueous composition is adjusted to be at least 6.

7. The process according to any of the preceding claims, **characterized in that** by the means of dewatering in step a) at least 70 percent of the water contained in the aqueous composition is removed.

8. The process according to any of the preceding claims, **characterized in that** by the means of dewatering in step a) the concentration of water is reduced to less than 1 percent by weight.

9. The process according to any of the preceding claims, **characterized in that** the means of dewatering in step a) is an azeotropic distillation with an additive, that forms a heteroazeotrope with water with a boiling point minimum.

10. The process according to any of claims 1 to 8, **characterized in that** the means of dewatering in step a) is an extractive distillation.

11. The process according to any of the claims 1 to 8, **characterized in that** the means of dewatering in step a) is a membrane separation method, using a semipermeable, hydrophilic membrane.

12. The process according to claim 11, **characterized in that** the membrane separation is combined with an additional distillation, where residual water contained in the retentate of the membrane separation is removed as a distillate and this distillate is returned to the membrane separation step.

13. The process according to claim 11 or 12, **characterized in that** the membrane separation is operated as a pervaporation with a liquid feed.

14. The process according to claim 11 or 12, **characterized in that** the membrane separation is operated as a vapor permeation with a gaseous feed.

15. The process according to claim 14, **characterized in that** the vapor permeation is combined with a pre-distillation in such a manner, that the vapor from the top of the pre-distillation column is directly contacted with the semipermeable membrane of the vapor permeation unit without prior condensation.

16. The process according to any of the preceding claims, **characterized in that** the means of distillation in step b) is a distillation method using a dividing-wall column
